(19) Europäisches Patentamt
European Patent Office
Office européen des brevets

(11) Publication number: **0 320 866**
**A2**

(12) **EUROPEAN PATENT APPLICATION**

(21) Application number: **88120779.9**

(22) Date of filing: **13.12.88**

(51) Int. Cl.4: **C07K 13/00 , C12N 15/00 , A61K 39/10**

(30) Priority: **18.12.87 IT 2308587**

(43) Date of publication of application: **21.06.89 Bulletin 89/25**

(84) Designated Contracting States: **AT BE CH DE ES FR GB GR LI LU NL SE**

(71) Applicant: **SCLAVO S.p.A.**
**Via Fiorentina 1**
**I-53100 Siena(IT)**

(72) Inventor: **Bartoloni, Antonella**
**Via delle Rose, 12 Località La Tognazza**
**I-53035 Monteriggioni (Siena)(IT)**
Inventor: **Pizza, Mariagrazia**
**Via dell'Oliviera, 23**
**I-53100 Siena(IT)**
Inventor: **Rappuoli, Rino**
**Via Calamandrei, 35**
**I-53010 Quercegrossa (Siena)(IT)**

(74) Representative: **Perani, Aurelio et al**
**c/o JACOBACCI-CASETTA & PERANI 7, Via Visconti di Modrone**
**I-20122 Milano(IT)**

(54) **A protective immunodominant epitope included in the S1 subunit of pertussis toxin.**

(57) There are described a protective immunodominant epitope of pertussis toxin which is included in the S1 subunit, and synthetic peptides with an aminoacid sequence similar to that of the epitope or to regions thereof which show an immunochemical reactivity with anti-PT protective monoclonal antibodiès equivalent to that of the protein itself. Ihe peptides are particularly useful for the preparation of an antipertussis vaccine.

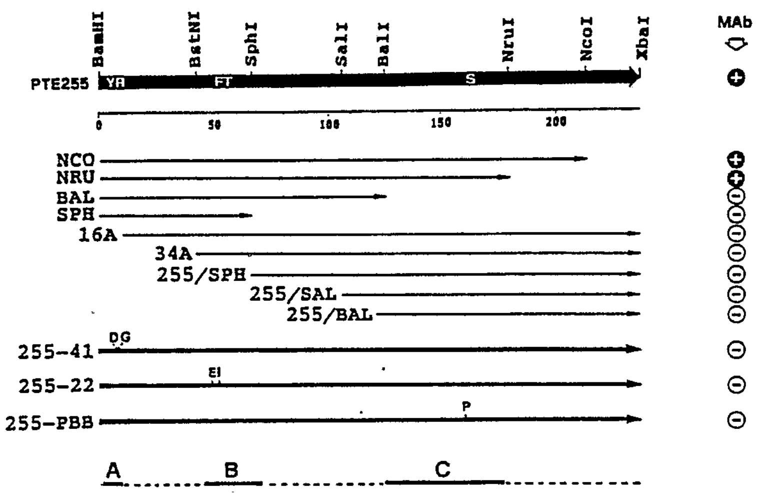


FIGURE 1

EP 0 320 866 A2

The present invention relates to an immunodominant epitope of pertussis toxin (PT) and, in particular, the protective immunodominant epitope included in the S1 subunit of PT. The invention also concerns synthetic peptides which have an identical aminoacid sequence to that of the epitope or to the regions thereof which show an immunochemical reactivity equivalent to that of the toxin itself towards the protective monoclonal antibodies against pertussis toxin.

The invention also concerns the use of the synthetic peptides for the preparation of a vaccine against pertussis and of diagnostic kits.

Pertussis is a disease of the respiratory tracts caused by Bordetella pertussis (B. pertussis) a bacillus which is transmitted aerogenically during the catarrhal and convulsive stages, from a sick person to a susceptible healthy individual.

Pertussis can cause convulsions, brain damage, and sometimes death, particularly in infants and in the newborn without maternal anti PT antibodies, and an effective vaccine against this disease is therefore particularly desirable.

A cellular vaccine against pertussis is currently used, which is constituted by virulent bacteria which have been killed with merthiolate and treated at 56° C, and whilst providing the individual with permanent protection, this is not completely satisfactory both because it causes undesirable side effects and because of the many problems deriving from its preparation, purification and stability.

There is thus a requirement to provide a vaccine against pertussis which does not have the disadvantages mentioned above.

There has been an increasing interest in recent years in the preparation of synthetic vaccines which do not contain intact natural antigens, but contain peptides which copy a very small region of an antigen, called an epitope or antigenic determinant, which can, however, induce the formation of antibodies which can neutralise the infectious agent.

It is known that epitopes may be contiguous, that it constituted by a particular linear aminoacid sequence, or discontiguous or confirmational, constituted by groups of aminoacids which are separated along the chain but which come together near a fold in the tertiary structure of the protein.

The preparation of a synthetic vaccine therefore requires both the protective antigen and the antigenic determinant (i) included in the antigen to be known, and in the case of conformational epitopes, requires the synthetic peptides which have a similar aminoacid sequence to that of the determinant to assume the same conformation as that presented in the natural antigen.

Up to the present time, the epitopes included in polysaccharide antigens have been studied widely, whilst very little is known about the structure and dimension of epitopes of protein molecules, and in particular, of pertussis toxin, which is a protein produced and secreted by B. pertussis in the virulent stage.

PT, which is constituted by five oligopeptides or subunits (S1, S2, S3, S4 and S5) whose genes have been cloned and sequenced as described and claimed in co-pending Italian patent application no. 19208 A/86, is one of the main anigens which are protective against infections caused by B. pertussis. Anti-PT antibodies have infact been found in individuals immunised with the cellular vaccine (Ashworth L.A.E. et al. (1983) Lancet Oct. 878-881) and effective protection has been achieved with the use of PT detoxified with formaldehyde in mice infected with virulent bacteria by aerosol means (Sato H, et al. (1983), Infect. and Immun. 41: 313).

The indentification of an immunodominant epitope of PT is therefore particularly desirable, both since it enables a better understanding of the mechanism by which the antigen acts, and since shorter peptides with a sequence identical to that of the epitope itself or to regions thereof are easier to synthesize and/or purify by conventional chemical synthesis methods or recombinant DNA techniques.

Of particular interest is the identification of the immunodominant epitope contained in the S1 subunit of pertussis toxin, which is constituted by 235 aminoacids. Sato et al (1987) (Infect. and Immun. 55, 909-915) in fact observed that monoclonal antibodies (MAbS) produced against the entire pertussis toxin and capable of neutralising PT in vitro and of protecting mice from intracerebral infections with virulent B. pertussis, recognize the enzymatically active S1 subunit.

According to the present invention, therefore, the regions of S1 which form the immunodominant epitope have now been identified and peptides have been synthesized, which contain one or more regions amd which, in vitro, are able to assume the correct conformation and to induce the formation of antibodies capable of neutralising native pertussis toxin.

In particular, three non-consecutive regions A, B and C, which are essential for the formation of the immunodominant epitope and, above all, for the formation of the correct conformation of this epitope, have been identified in the peptide chain of S1, between the aminoacids 1-20, 40-80 amd 123-180 respectively.

Moreover, it has been found that synthetic peptides containing only one of these regions are able to refold themselves again with peptides contain one or both the other two regions, into the correct conformation in vitro.

Again, it has been found that the substitution of an aminoacid in one of these regions destroys the ability of the peptides to assume the necessary configuration for binding the antibodies.

An object of the present invention is therefore to localise and identify the immunodominant epitope contained in the S1 subunit of pertussis toxin.

Another subject of the present invention is constituted by synthetic peptides with an aminoacid sequence identical to that of the immunodominant epitope of S1 and/or to regions thereof which can induce the formation of antibodies which can neutralise the natural antigen.

A further subject of the present invention is a vaccine containing a therapeutically-effective quantity of the peptides.

A subject of the present invention is also constituted by the use of the synthetic peptides for the preparation of diagnostic kits for the determination of infections caused by B. pertussis. Further objects of the present invention will become clear from a reading of the text and from the examples which follow.

According to the present invention, therefore, the immunodominant epitope contained in S1 was determined by the testing of the immunoreactivity towards anti-PT monoclonal antibodies of synthetic peptides which have an aminoacid sequence like that of S1 but without aminoterminal and/or carboxyterminal regions of different lengths.

Ihe synthetic peptides can be prepared by chemical synthesis by one of the generally-known techniques, or by means of a fermentation method which uses a host micro-organism transformed by a hybrid plasmid containing the gene which codes for the S1 subunit without the 3′ terminal and/or 5′ terminal nucleotide sequences of different lengths.

In particular, the synthetic peptides were prepared by means of a method which comprises the digestion of the PTE 255 plasmid described and claimed in Italian patent application no. 19208 A/86 with EcoRI and HindIII restriction enzymes, the separation from the digestion mixture of the 600 base-pair (bp) DNA fragment containing the genes which codes for S1, the digestion of the fragment with suitable restriction enzymes, the ligation of the resulting fragments without the 3′ terminal and/or 5′ terminal nucleotide sequence, in the presence of T4 DNA ligase enzyme, to a previously-linearised expression plasmid and finally the8 transformation of a host micro-organism by the hybrid plasmid.

Host micro-organisms suitable for the purpose may be selected from Escherichia coli (E. coli), Bacillus or yeasts.

In particular the commercially available K2, ΔHI,Δtrp E.coli strain (Remaut, E. et al. (1981) Gene, 15 : 81-93) was used.

The transformed strains were then grown in a suitable culture medium in the presence of sources of carbon, nitrogen and mineral salts, at a temperature of from 30 °C to 45 °C for a period of from 2 to 18 hours.

The cells were then separated from the culture medium and lysed by means of generally known techniques, and the peptides were recovered and purified from the lysed cells.

In accordance with the present invention, hybrid plasmids were constructed which enable the expression of the following synthetic peptides (Figure 3):

NCO (1-212) without the 213-235 carboxyterminal sequence;
NRU (1-180) without the 181-235 carboxyterminal sequence;
BAL (1-123) without the 124-235 carboxyterminal sequence;
SAL (1-109) without the 110-235 carboxyterminal sequence;
SPH (1-68) without the 69-235 carboxyterminal sequence;
255/SPH (68-235) without the 1-67 aminoterminal sequence;
255/BSL (124-235) without the 1-123 aminoterminal sequence;
255/SAL (109-235) without the 1-108 aminoterminal sequence;
16-A (11-235) without the 1-10 aminoterminal sequence;
18-A (149-235) without the 1-148 aminoterminal sequence;
34-A (53-212) without the 1-52 aminoterminal sequence and the 213-235 carboxyterminal sequence;
NCO-BAL (124-212) without the (1-123) aminoterminal sequence or the (213-235) carboxyterminal sequence.

The peptides were then tested by Dot blot (Burnette, W.N. (1981) Anal. Biochem. 112, 195-203) and radioimmunoassay to determine their immunoreactivity towards anti-PT monoclonal antibodies.

Antibodies suitable for the purpose may be selected from those known in the art, and in particular from the protective and non-protective ones which react with the S1 subunit.

Preferably used were the protective MAbs L10 (Perera, V.Y., et al J. Gen. Microbio. 132, 553-556 (1986)), 3F10 and 1B7 (Sato H. et al. (1984) Infect Immun. 46, 422-428; Sato H. et al. (1987) Infect Immun. 55, 909-915).

2, 3, 4 and 5 obtained by Drusilla Burns and C. Manclark and the non-protective MAbs (obtained by Drusilla Burns and C. .Manclark) and 6G7 (obtained in our laboratory according to generally-known techniques).

From the data shown in Table 1 and in Figure 1, the following can be observed:

The 6G7 Mab (non-protective) recognises all the peptides except BAL, SAL, SPH and 18-A so that it must map the region of S1 between the aminoacids 123 and 149.

Since the monoclonal antibody does not recognise the S1BPP subunit of parapertussis toxin, which has a threonine in position 143 instead of arginine (Arg), it may be concluded that the presence of this aminoacid is essential because the peptide was recognised.

The 1 MAb (non-protective) recognises all the peptides which lack the carboxyterminal aminoacid region including SPH (1-68) and does not recognise the peptide 16-A which lacks the first 10 aminoacids. It may therefore be concluded that the region of S1 involved is between the aminoacids 1 and 68 and that the aminoacid sequence 1-10 is particularly important in the structure of the epitope.

The 2, 3, 4, 5, 3F10, 1B7 and L10 MAbs (protective) recognise the NCO and NRU peptides but do not recognise BAL, SAL and SPH do that they must map the region of S1 between aminoacids 180 and 123. Since L10 recognises neither the S1BPP subunit nor the S1BB subunit of bronchiseptic toxin which contains Proline (Pro) in position 162 instead of serine (Ser), it was thought that it was the region around this position which was recognised by the anti-PT monoclonal antibodies.

However, this theory was discarded when it was observed, suprisingly, that peptides whichlack the aminoterminal sequence but include the 124-180 region did not give an immunochemical reaction. It was therefore obvious that the sequence upstream of the aminoacid in position 123 forms part of the epitope recognised by the MAbs.

Since the antibodies did not recognise the 16-A peptide which lacks the aminoterminal sequence of 10 aminoacids, it was concluded that both the region 1-20 (region A-Figure 1) and the region 124-180 (region C in Figure 1) were necessary for binding the antibodies.

In order to check whether the two halves of the S1 molecule, which were not recognised separately by the monoclonal antibodies, could reconstitute the immunodominant epitope *in vitro*, the peptides BAL (1-123) and PH (1-68) were mixed with each of the peptides lacking the aminoterminal sequence which had given negative immunoreactions. In particular, the peptides were made soluble in 8M urea with the addition of 5 mM mercaptoethanol and the resulting mixtures were reacted at 4°C for a period of from 4 to 24 hours. The mixtures were then dialysed for 24 hours against saline phosphate buffer (SPB) containing 4M urea and for 72 hours against SPB containing 2M urea.

The mixtures were then suitably diluted and used to determine their immunoreactivity towards the MAbs by means of Dot blot analysis and radio-immunoassays. As shown in Table 2, the peptide BAL, which alone was not recognised by the monoclonal antibodies when mixed with the peptides lacking the aminoterminal sequence, gave a positive reaction.

This indicated that the two halves of S1 were capable of assuming the correct confirmation in order to be recognised by the antibodies and that the regions around the aminoacid in position 123 were not necessary for the formation of the epitope. In fact a mixture of the two peptides BAL and 255/BAL, whch had been broken in this region, was recognised by the monoclonal antibodies.

The results obtained by the testing of the mixtures containing the peptide SPH and each of the peptides lacking the aminoterminal region showed, however, that SPH could be complemented only by the pepties 16-A and 34-A.

This suggested that a third region in the vicinity of the position 68 (region B in Figure 1) was necessary for the formation of the epitope and for the binding of the monoclonal antibodies.

In order to confirm what had been observed, that is that the regions A, B and C were necessary for the formation of the immunodominant epitope and therefore for the binding of the antibodies, the gene which codes for the S1 subunit was modified by site-directed mutagenesis which enables mutations to be introduced very precisely into sites of a DNA molecule *in vitro* and the effect of the mutation to be tested *in vitro* and *in vivo*.

By this method it is possible to replace the required bases using one of the following methods:

- by the incorporation of analogues of bases in sites of the DNA;
- by the incorrect incorporation of nucleotides;
- by the introduction of a mutation during the synthesis *in vitro* of oligonucleotides with defined sequences;
- by the use of specific muagenic chemicals, such as sodium bisulphite, which react with the bases of the DNA.

According to the present invention, the gene which codes for S1 was modified by the use of synthetic oligonucleotides with defined sequences according to the method described by Zoller M.J. et al, (DNA 3:479-488, (1984)).

In practice, the 600 bp EcoRI-HindIII DNA fragment derived from the clone PTE 255 was cloned in a vector which enables the isolation of the DNA of the fragment cloned in a single chain.

Vectors suitable for the purpose may be selected from Bluescript SK (Stratagenes. Diego, C.a.), pEMBL (Dante et al Nucleic Acids Research 11, 1645-1655 (1983) or M13 bacteriophases (Viera an Messing (l982) Gene, 19, 263).

According to the present invention the commerically available vector Bluescript SK was used.

This vector was treated with Ecorl and HindIII restriction enzymes and then ligated to the 600 bp DNA fragment in a ligation mixture in the presence of the T4 DNA ligation enzyme.

The mixture was then used to transform commercially available E.coli JM101 cells and the transformants were subsequently selected on an LB agar culture medium with the addition of 100 µg/ml of ampicillin.

The positive clones, that is those containing the hybrid plasmids constituted by the vector and the 600 bp DNA fragment were suspended in a liquid medium in the presence of bacteriophages and kept at a temperature of from 30° to 40° C for a period of from 2 to 10 hours.

At the end of this period, the bacteriophages were precipitated, separated from the solution by centrifuging, resuspended in a pH 7.5 buffer, extracted with saturated phenol with water and ethyl ether and finally with ethanol and ammonium acetate to precipitate the single-strand DNA.

Portions of the DNA were then used to modify the S1 gene by direct mutagenesis. For this purpose, oligonucleotides of approximately 20 nucleotides were synthesised, in which the bases which code for one or were aminoacids present in particular sites of the 1-180 region of S1 were replaced by others which code for a different aminoacid. In particular the oligonucleotides were synthesised, which enable the following mutants of the gene coding for S1 to be prepared:

41: Tyrosine 8 and arginine 9 were replaced respectively by aspartic acid and glycine, using the primer GTCATAGCCGTCTACGGT..
The corresponding gene was modified thus:
620-CGCCACCGTATACCGCTATGACTCCCGCCCG-650
620-CGCCACCGTAGACGGCTATGACTCCCGCCCG-650

22: Phenylalanine 50 and threonine 53 were replaced respectively by glutamic acid and isoleucine using the primer TGGAGACGTCAGCGCTGT. The corresponding gene was modified thus:
The sequence 750-AGCGCTTTCGTCTCCACCAGC-770 was changed into 750-AGCGCTGACGTCTCCATCAGC-770.

25: Glycine 99 was replaced by glutamic acid using the primer CTGGCGGCTTCGTAGAAA;
The corresponding gene was modified thus:
the sequence 910-TACGGCGCCGC-920 was changed into 910-TACGAAGCCGC-920.

17: Aspartic acid 109 was replaced by glycine using the primer CTGGTAGGTGTCCAGCGCGCC.
The corresponding gene was modified thus:
the sequence 930-GTCGACACTTA-940 was changed into 930-GTCGGCACTTA-940.

27: Glycine 121 was replaced by glutamic acid using the primer GCCAGCGCTTCGGCGAGG.
The corresponding gene was modified thus:
the sequence 956-GCCGGCGCGCT-966 was changed into 956-GCCGAAGCGCT-966.

16: Alanine in position 124 was replaced by aspartic acid using the primer GCCATAAGTGCCGACGTATTC
The corresponding gene was modified thus:
the sequence 976-TGGCCACCTAC-984 was changed into 976-TGGACACCTAC-986.

1716: contains the two mutations 16 and 27 combined.

28: glutamic acid 129 was replaced in glycine using the primer GCCAGATACCCGCTCTGG.
The corresponding gene was modified thus:
the sequence 990-AGCGAATATCT-1000 was changed into 990-AGCGGGTATCT-1000.

29: Arginine 135 was replaced by glutamic acid using the primer GCGGAATGTCCCGGTGTG.
The corresponding gene was modified thus:
the sequence 1010-GCGCATTCCGC-1020 was changed into 1010-GGACATTCCGC-1020.

31: Threonine 159 was replaced by lysine using the primer TACTCCGTTTTCGTGGTC.
The corresponding gene was modified thus:
1070-GCATCACCGGCGAGACCACGACCACGGAGTA-1090 was changed into 1070-GCATCACCGGCGAGACCACGAAAACGGAGTA-1090.

26. Tyrosine 111 was replaced by glycine. Moreover, because of a partial duplication of a fragment of the primer, the aminoacids Aps Thr Gly Gly were inserted in position 113 using the primer CGCCACCAGTGCGACGTATTCGA.

The corresponding gene was modified thus:

930-GTCGACACTTATGGCGACAAT-950

930-GTCGACACTGGTGGCGACACTGGTGGCGACAAT-950

The oligonucleotides were used as primers for the DNA polymerase which copies the whole of the nucleotide sequence of the vector incorporating the mutations present therein.

The vectors containing the S1 gene with the required modification were isolated by the hybridisation technique using the primer itself as the probe.

The exact nucleotide sequence of the modified gene was then confirmed by the technique of Sanger F. et al (P.N.A.S 74, 5463, 1977).

The vectors containing the modified genes were then digested with EcoRI and HindII restriction enzymes and the DNA fragments containing the gene which codes for modified S1 were cloned in an expression plasmid selected from those known in the art.

The hybrid plasmids were used to transform a host micro-organism selected from E. coli, Bacillus subtilis and yeasts.

In particular, according to the present invention, the plasmid Pex-34 (Center for Molecular Biology, Heidelberg, Federal Republic of Germany) and the micro-organism E. coli K12-ΔH1-Δtrp (Remaut, E. et al, Gene, 15, 81-93, 1981) were used.

The transformed micro-organisms were then grown in liquid culture mediums in the presence of sources of carbon, nitrogen and mineral salts, at a temperature between 30° and 45° C for a period of from 20 minutes to 5 hours.

Upon completion, the cells were recovered from the culture medium by centrifuging and lysed accoridng to generally known methods.

The lysed cells, containing the mutated peptides, were then analysed to determine their immunoreactivity towards the L10 monoclonal antibody.

In this way three peptides (41, 22 and PBB) were isolated which were not recognised by the L10 MAb and each of the three contained mutations in the regions A, B and C (Figure 2) respectively. This confirmed that these regions were essential for binding with the L10 antibody and that a mutation in only one of these regions would prevent the MAb from recognising the epitope. According to the present invention, the three mutants were tested by means of Dot blot to determine their immunoreactivity towards the other protective MAbS and it was found that they were capable of recognising the peptides 22 and PBB, but not 41. This indicated that whilst the three regions A, B and C were necessary, since the epitope reassumes the correct conformation and is therefore recognised by the MAbS, the specificity of the latter varied within these regions.

According to the present invention, therefore, synthetic peptides containing the three non-contiguous regions A, B and C can be prepared by means of conventional chemical synthesis techniques or by recombinant DNA techniques.

According to another embodiment of the present invention, the peptides can be prepared by the synthesis of shorter peptides containing one or two of the regions and mixing them in vitro under conditions such as to enable them to refold again in the correct conformation.

According to the present invention, the mechanism by which the S1 subunit acts were also identified.

lhe subunit contains two cysteines in positions 41 and 201 which are joined by a disulphide bridge and in order to be enzymatically active (toxic) this bridge be broke.

The monoclonal antibodies described above bind the two sequences near the disulphide bridge and prevent the molecule from moving the regions containing the two cysteins apart and thus becoming active.

This explains why the S1 subunit produced by the enzymatically active clone 255 does not induce good protection.

In fact, it is expressed in the form of a polypeptide without the disulphide bridge and only a few molecules have the correct conformation to bind the antibodies.

The enzymatically activesubunit S1 obtained by means of the fermentation of a strain transformed by the plasmid PTE 255 can therefore be treated suitably so as to produce molecules with the correct conformation and the molecules can therefore be used for the preparation of a vaccine against pertussis.

The present invention also relates to a method for inducing immunity against pertussis, which comprises the administration, in man, of an immunologically-effective quantity of a peptide of the present invention or a mixture thereof of such peptides.

The therapeutically effective quantity can easily be determined by experts in the art and is generally between approximately 0.1 ug/kg and approximately 100 µg/kg of body weight.

The dose is preferably between 0.1 and 1.0 µg/kg of body weight.

The method of administration of the peptides may be selected from those which are most suitable and which at any rate put the peptides into contact with the immune system.

In particular the peptides may be administered intramuscularly, intravenously, or by any other method which enables the active agents to reach the lymphocytes and induce an immune response.

The peptides of the present invention may be used for the preparation of pharmaceutical compositions containing the active agent in a form suitable to induce an immune response.

Aqueous solutions or suspensions containing the peptides in a form which is easy to inject are preferable.

Adjuvants may be used in the preparation of the compositions to increase the immune response to the peptides.

According to the present invention, the peptides may be administered to man either alone or bound to a macromolecular support selected from those generally used in the art to increase the immune response to an antigen.

In all cases the structure of the support is not fundamental since its capacity to increase the immune response depends on its dimensions.

When macromolecular supports such as proteins and polysaccharides are used, they are preferably compounds with a molecular weight of between 10,000 and 1,000,000.

The method for binding the peptides to a support can be selected from those known in the art and must at any rate be such as not to cause the loss of the immunising capacity of the peptide.

According to the present invention, the synthetic peptides may be used for the preparation of a diagnostic kit for the determination of infections caused by B. pertussis in clinical samples.

## Brief description of the drawings

Figure 1: a map of the S1 subunit of pertussis toxin and of the mutants.
The arrows indicate the part of the S1 molecule which is expressed by each clone.
The aminoacids combined in the mutants are indicated 155-41, 255-22 and 255-PBB; the corresponding aminoacids in the molecule of the natural strain are shown as white letters within the PTE 255.
The numbers shown under the PTE 255 clone refer to the aminoacid sequence.
On the right the reactivity (+ or -) with the monoclonal antibodies shown by each mutated protein upon Western blotting is given.
The reactivity of the MAbs refers to the seven MAbs tested, except for 255-22 and 255-PBB, which were not recognised only by MAb L10.

Figure 2: The Dot Blot analysis is given which shows that the L10 antibody does not recognise the BAL (1A) and SPH (2A) mutants lacking carboxyterminal regions or the 34A (1B and 2B) mutant lacking aminoterminal region. After refolding in vitro mixtures of the BAL and 34A (1C and SPH and 34A (2C)) mutants are recognised by the antibody.
1D and 2D contain the whole S1 molecule expressed by the PTE 255 clone (control).

Figure 3: The sequences are given of the S1 subunit of Bordetella pertussis (S1BP), of Bordetella parapertussis (S1BPP) and of Bordetella bronchiseptica (S1BB).
Also given (broken lines) are the sequences of the various mutants obtained by direct-site mutations and deletions of the S1 gene.

The following examples are illustrative and not limiting of the invention itself.

## Example 1

### A. Construction of the hybrid plasmids containing the gene which codes for S1 modified by the deletion of the 3 terminal part

10 µg if the plasmid PTE 255 were suspended in 100 µl of buffer solution (50mM Tris-HCl, pH 7.4, 10 mM $MgC1_2$, 100 mM MaCl) and digested at 36°C for 2 hours with 30 units (U) of Xbal (BRL) restrictions enzyme and 10 µl portions of the digestion mixture were then treated with 3U of one of the following enzymes: Mcol, Ball, Nrul, Sall and Sphl at 37°C for another two hours.

3U of large-fragment Klenow polymerase and 2 µl of a solution containing 50mM of each of the following desoxynucleotides, ATP, dTTP, dCTP and dGTP were added to the DNA mixtures thus digested containing the 75 base-pairs (bp) Xbal-Ball, 377 pp Xbal-Ball, 165 bp Xbal-Nrul, 355 bp Xbal-Sall and 503 bp Xbal-SphI fragment respectively to fill in the ends of the molecules. The mixtures were kept at ambient temperature (20-25° C) for 15 minutes and at 65° C for another 30 minutes as to render the polymerase enzyme inactive. At the end of this period, the mixtures were diluted to 200 µl with ligation buffer (66mM Tris-HCl, pH 7.6, 1mM ATP, 10 mM $MgCl_2$, 10 mM Dithiothreitol) and kept at 15° C for one night in the presence of 1U of T4 DNA ligase so that the DNA molecules which had lost the fragments given above recombine. The ligation mixtures were then used to transform E. coli K12-ΔH1Δtrp cells made competent by treatment with 50 mM $CaCl_2$ (Mandel M and Higa (1970) I. Mol. Biol. 53, 154).

The selection of the transformants was carried out by placing the cells on LB agar medium plates (10 g/1 Bacto Tryptone (DIFCO), 5 G/l Bacto Yeast extract (DIFCO) 5 g/l NaCl) with the addition of 30 ug.ml of ampicillin and incubating the plates at 30° C for 18 hours. The recombinant plasmids were analysed so as to check the exact nucleotids sequence.

The following hybrid plasmids were thus identified:

PTE NCO in which the S1 gene lacks the part which codes for the carboxyterminal sequence between the aminoacids 235 and 213 of the S1 subunit and codes for a peptide 1-212.

PTE NRU in which the S1 gene lacks the part which codes for the carboxyterminal sequence between the aminoacids 235 and 181 of the S1 subunit and codes for a peptide 1-180.

PTE BAL in which the S1 gene lacks the part which codes for the carboxyterminal sequence from 235 to 124 of the S1 subunit and codes for a peptide 1-123.

PTE SAL in which the S1 gene lacks the part which codes for the carboxyterminal sequence between the aminoacids 235 and 110 of the S1 subunit and codes for a peptide 1-109.

PTE SPH in which the S1 gene lacks the part which codes for the carboxyterminal sequence between the aminoacids 235 and 69 of the S1 subunit and codes for a peptide 1-68.

B. Construction of the hybrid plasmids containing the gene which codes for S1 modified by the deletion of the 5 terminal part

3 samples (10 µg) of the plasmid PTE 255 were digested in 100 µl of a buffer solution (50 mM Tri-HCl pH 7.4, 10 mM $MgCl_2$, 50 mM NaCl) at 37° C for 3 hours with 30 U of one of the following restriction enzymes SphI, SalI and BalI respectively. 3 U of the large-fragment Klenow polymerase enzyme and 2 µl of a solution containing 50 mM of each of the following desoxynucleotides: dATP, dTTP, dTTP and dGTP were then addd to each solution and after 15 minutes at 20-25° C, the enzyme was rendered inactive at 65° C for 30 minutes. 30 U of HindIII restriction enzyme was then added to each solution and the resultant mixtures were kept at 37° C for 3 hours and then loaded onto 1.5% agarose gel and run at 70 volts for 3.5 hours. Two bands were then separated for each mixture, one containing the deleted part of S1, and the other containing the plasmid PeX-34 and part of S1.

The 520-bp Sph-HindIII, 372-bp SalI-HindIII and 394-bp BalI-HindIII fragments were then electroeluted by Maniatis' method (Molecular Cloning: A Laboratory Manual, Cold Spring Harbor,1982). 100 ng of each of the fragment were then ligased in 30 ul of ligation mixture in the presence of 1 U of T4 DNA ligase, with the plasmid Pex-34 previously digested with BamHI restriction enzyme treated with the polymerase enzyme and a solution of desoxynucleotides and then digested with HindIII restriction enzyme.

The ligation mixtures were then used to transform E. coli K12, ΔHI,Δtrp cells and the transformants were selected on LB agar medium with the addition of ampicillin as described in section A.

From the plasmids extracted from the positive clones, those containing the cloned fragments in the correct reading frame were identified by means of Western blot with anti-pertussis toxin antibodies. lhe plasmids, indicated by the symbols PTE 255/SPH, PTE 255/SAL and PTE 255/BAL lacked the sequences of the S1 gene which code for the aminoterminal part of the subunit between the aminoacids; 1-67, 1-108 and 1-123 respectively thus coding for the peptides 68-235, 109-235 and 124-235.

C. Construction of the hybrid plasmid containing the gene which codes for S1 modified by the deletion of the 3 and 5 terminal parts.

2 samples (10 µg) of the plasmid PTE NCO (obtained as described in step A) were digested in 100 µ1 of a 50 mM Tris-HCl, pH 7.4, 10 mM $MgCl_2$, 50 mM NaCl solution with 30 U of BstN1 (BRL) and with 30 U

of Ball (BRL) respectively at 37° C for 3 hours.

The digestion mixtures were then treated at 20-25° C for 15 minutes with 3 U of Klenow polymerase enzyme in the presence of 2 mM of dATP, dGTP, dCTP and dTTP to fill the terminals and after the enzyme had been rendered inactive at 65° C for 30 minutes, the DNAS were again cut with 30 U of HindIII restriction enzyme at 37° C for 3 hours.

The digestion mixtures were loaded onto 1.5% agarose gel and run at 70 volts for 3.5 hours and the 527-bp BstNI-HindIII and 279-bp Ball-HindIII fragments were electroeluted as described above. 100 ng of the fragments were then ligased to the plasmid Pex-34, (previously treated as described in B) in a ligation mixture in the presence of T4 DNA ligase at 15° C for 18 hours. E. coli cells were then transformed and the transformants selected as described above. From the recombinant plasmids extracted from the positive clones, those having the fragments inserted in the correct reading frame were identified.

The plasmids, indicated by the symbols PTE 34A and PTE NCO/BAL contained respectively the S1 gene lacking the sequences which code for the parts of the S1 subunit between the aminoacids 1-52 and 235-213 and for the parts 1-123 and 235-213 and code respectively for the peptides 53-212 and 124-212.

D. Construction of the plasmid PTE 16-A and PTE 18-A

10 µg of the plasmid PTE 255 were digested in 100 µl of a 100 mM Tris-HCl, 50 mM MaCl, 10 mM $MgSo_4$ buffer with 30 U of EcoRI and then with 1 U of Ba131 (BRL) in 10 mM $CaCl_2$, 10 mM $MgCl_2$, 0.2 M NaCl, 20 mM Tris-HCl, pH 8.1 mM EDTA at 37° C. Portions of the mixture were withdrawn after 1, 3, 5 and 10 minutes and the fragments deleted at the 5′ terminal were then cut with HindIII, purified by means of gel electrophoresis and after elution, ligased to the plasmid Pex-34 as described above. The ligation mixtures were then used to transform E. coli cells and the transformants were selected as described in the preceding steps.

From the plasmids extracted from the positive clones, those containing the fragments of the S1 gene inserted in the correct frame, determined by the analysis of their nucleotide sequences, were isolated.

From the various plasmids thus obtained, those containing the S1 gene without the sequence which codes for aminoterminal part of S1 were selected.

In particular, the plasmid PTE 16-A lacks some nucleotides which code for the first 10 aminoacids and therefore codes for a peptide containing the aminoacids 11-235, whilst the plasmid PTE 18-A codes for a peptide containing the aminoacids 149-235.

Example 2

Expression of the modified S1 subunits and determination of their immunochemical reactivity towards anti-PT monoclonal antibodies

A.

E. coli K12, Δ H1,Δtrp cells transformed by the plasmids prepared as described in Example 1 above were grown in 20 ml of LB liquid medium with gentle agitation at 30° C for 1 night. 10 ml of each culture were used to innoculate 400 ml of LB medium and grown at 30° C for 2 hours and at 42° C for 2.5 hours.

At the end of this period, the cultures were centrifuged at 10,000 rpm for 15 minutes at 4° C, the supernatant liquids were discarded, the cells were recovered and resuspended in 3.2 ml of a solution of 2.5% sucrose, 10 mM Tris-HCl (pH 8.0), 1 mM EDTA.

0.1 ml of a solution of lysozyme (40 mg/ml) and 0.8 ml of 0.5 M EDTA were then added to the solutions and reacted at 37° C for 30 minutes.

8 ml of a lysing buffer (1% Triton-X 100, 50 mM Tris-HCl, pH 6.0, 63 mM EDTA) were then added to each solution and kept at 0° C for 15 minutes and at 37° C for 30 minutes.

After sonication for 1 minute, the mixtures containing the lysed cells and the inclusion bodies were centrifuged at 10,000 rpm for 10 minutes, the supernatant liquids discarded and the precipitates resuspended in 5 ml of 1M urea and kept at 37° C for 30 minutes.

The mixtures were centrifuged again and the precipitates or inclusion bodies recovered and made soluble in 5 ml of saline phosphate buffer (SPB) and kept at -20° C.

Before proceeding with the Dot blot test, 100 µl of the solutions containing the inclusion bodies were centrifuged and the precipitates made soluble for one night in 100 µl of 8M urea containing 10 mM of dithiothreitol (DTT).

10µl of each suspension (1 µg of peptide) were then diluted 1/10 in SPB and immediately loaded on to a mitrocellulose filter using a minifold apparatus (Schleicher and Shull, Dallselt, Germany).

The filters containing the peptides which had been made insoluble were then suspended in 0.15 M NaCl, 10 mM phosphate pH 7.4 SPB containing 1% Denhart solution (0.03% bovine albumin, 0.2% Ficoll 70 and 0.02% polyvinylpyrrolidone) and 0.05 Tween 20 and kept at ambient temperature for 2 hours.

They were then washed twice (15ml x 3 minutes) with PBS with the addition of 0.05 of Tween 20 and incubated for one night at ambient temperature (20-25°C) with 10 ml of SPB with the addition of 0.05% Tween 20 containing the monoclonal antibodies (4 µg/ml).

The filters were washed again (3 x 15 minutes) with 10 ml of 10 mM Tris, 0.9% NaCl and 0.1% Tween 20 (SPB) solution and incubated with goat gammaglobulin anti-mouse-gammaglobulin conjugated with peroxidase diluted 1/100 in TBS and finally washed three times in TBS and once in 0.05 M Tris pH 6.8, 5 ml 0.3% 4-chloro-1-naphthol in methanol and 7 µl of $H_2O_2$.

After 5-60 minutes at ambient temperature, the filters were washed in distilled water to stop the enzymatic reaction. As can be seen from the results given in Table 1 below, the 1-10 regions and the region downstream of the aminoacid in position 123 are essential for the formation of the immunodominant epitope.

Moreover, the replacement of a serine in position 163 by proline (S1BPP and S1BB) is not recognised by the L10 antibody.

Table 1

| Synthetic peptides | Dot Blot Analysis | | | | | | | | | |
|---|---|---|---|---|---|---|---|---|---|---|
| | ANTIBODIES | | | | | | | | | |
| | PROTECTIVE | | | | | | | | NON-PROTECTIVE | |
| | L10 | 3F10 | 1B7 | 2 | 3 | 4 | 5 | 6 | 1 | 667 |
| S1255 | + | + | + | + | + | + | + | + | + | + |
| S2BPP | - | + | + | + | + | + | + | + | + | + |
| S1BB | - | + | + | + | + | + | + | + | + | + |
| NCO(1-212) | + | + | + | + | + | + | + | + | + | + |
| NRU(1-180) | + | + | + | + | + | + | + | + | + | + |
| BAL(1-123) | - | - | - | - | - | - | - | - | + | - |
| SAL(1-109) | - | - | - | - | - | - | - | - | + | - |
| SPH(1-68) | - | - | - | - | - | - | - | - | + | - |
| 16-A(11-235) | - | - | - | - | - | - | - | - | - | + |
| 34-A(53-212) | - | - | - | - | - | - | - | - | - | + |
| 255/BAL(124-235) | - | - | - | - | - | - | - | - | - | + |
| 255/SAL(109-235) | - | - | - | - | - | - | - | - | - | + |
| 255/SPH(68-235) | - | - | - | - | - | - | - | - | - | + |
| NCO/BAL(124-212) | - | - | - | - | - | - | - | - | - | + |
| 18-A(149-235) | - | - | - | - | - | - | - | - | - | - |

S1-255 S1 subunit of 235 aminoacids obtained by the expression of the plasmid PTE 255

S1 BPP S1 subunit of Bordetella pertussis

S1BB subunit of Bordetella bronchiseptica toxin

B) Dot blot test and radioimmunoassay of peptides rewound in vitro.

1 volume of a solution containing the peptide SPH and 1 volume of a solution containing the peptide BAL were mixed respectively with 1 volume each of the mutants containing the aminoterminal deletions and then dialysed for 24 hours against 100 volumes of 4M urea and for 72 hours against 100 volumes of 4M urea and saline phosphate buffer. Ihe peptides thus folded were kept at 4° C in 2M urea until the moment of use (1µg/ml).
The reactivity of the refolded peptides towards the monoclonal antibodies was then tested by Dot blot testing as described in step A) and radioimmunoassay (RIA).

In practice, the RIA test was carried out as follows: the peptides were diluted 1/10 in SPB and 50 µl were introduced on to microtitration plates, the diluent being used alone as a control, and 50 ul of monoclonal antibody in SPB and 50 ul of goat anti-mouse-γ-globulin globulin marked with $I^{125}$ (≈100,000 cpm per chamber) were added.

The plates were incubated at 4° C for 18 hours, washed 5 times with SPB-BSA-$NaN_3$ and then the radioactivity present in each chamber was determined in a gamma counter. From the results given in Table II it can be seen that the peptide 1-123 is able to assume with all the other peptides in vitro the correct configuration to be recognised by all the protective monomclonal antibodies, whilst the peptide SPH (1-68) gives an exact refolding only with the peptides 16-A (11-235) and 34-A (53-212). This indicates that a third region present in the refion around the position 68 is essential for the formation of the immunodominant epitope and therefore for recognition by the MAbs.

Table II

| MONOCLONAL ANTIBODIES | | | | | | | | |
|---|---|---|---|---|---|---|---|---|
| peptides | Dot blot | | | | | | | Radioimmunoassay (cpm) |
| | L10 | 3F10 | 1B7 | 2 | 3 | 4 | 5 | 2 |
| SPH | - | - | - | - | - | - | - | |
| + | | | | | | | | |
| 16-A | + | + | + | + | + | + | + | 5760 |
| 34-A | + | + | + | + | + | + | + | 5880 |
| 255 SPH | + | + | + | + | + | + | + | 526 |
| 255/SAL | - | - | - | - | - | - | - | 346 |
| 255/BAL | - | - | - | - | - | - | - | 393 |
| NCO/BAL | - | - | - | - | - | - | - | 393 |
| 18-A | - | - | - | - | - | - | - | 422 |
| Bal | - | - | - | - | - | - | - | 407 |
| + | | | | | | | | |
| 16-A | + | + | + | + | + | + | + | 6030 |
| 34-A | + | + | + | + | + | + | + | 5490 |
| SPH/HIND | + | + | + | + | + | + | + | 5344 |
| 255/BAL | + | + | + | + | + | + | + | 7592 |
| 255/BAL | + | + | + | + | + | + | + | 6700 |
| NCO/BAL | + | + | + | + | + | + | + | 372 |
| 18-A | + | + | + | + | + | + | + | 325 |

The results of the radioimmunoassay, obtained with the monoclonal antibody 2, reflect identical results obtained with the other protective monoclonal antibodies.

## Example 3

### Preparation of mutants of the S1 gene

10 µg of the plasmid PT 255 were suspended in 100 µl of 10 mM Tris-HCl, pH 7.5, 50 mM NaCl, 10

mM $MgCl_2$ buffer and digested with 30 U of each of the EcoRI and HindIII enzymes at 37° C for 3 hours.

The digestion mixture was then loaded on to 1.3% agarose gel and run at 80 mA for 3 hours.

Two bands were thus separated; one of 3500 bp containing the vector, and the other of 600 bp, containing the gene which codes for the S1 subunit. The 600 bp band was then electroeluted and 0.2 µg of the fragment and 0.3 ng of the plasmid Bluescript SK (Stratagene, San Diego, Ca), previously digested with the EcoRI and HindIII restriction enzymes, were suspended in 20 µl of buffer (66 mM Tris-HCl, pH 7.5, 1mM ATP, 10 mM $MgCl_2$, 10 mM dithiothreitol) and ligated in the presence of 1 U of $T_4$ DN ligase at 15° C for 18 hours.

The ligation mixtures was then used to transform E. coli JM 101 cells which had been made competent and the transformants were selected on LB agar plates with the addition of 100 µg/ml of ampicillin, 20 µg/ml of ITPG (isopropyl-B-D-thiogalactopyranoside) and 20 µg/ml of X-Gal (5-bromo-4-chloro-3-indolyl D-galactopyranoside). Ihe plates were incubated at 37° C in a thermostatically-controlled chamber for 18 hours. The white colonies containing the hybrid plasmid constituted by the vector Bluescript SK and by the 600-bp DNA fragment were used to isolate the single chain DNA of the cloned fragment as follows. The colonies were grown in 1.5 ml of LB liquid medium to an optical density (OD) at 590 mm of approximately 0.15.

10 µl of a suspension of the bacteriophage F1 (Stratagene, San Diego, Ca) in LB (5 x $10^{12}$ bacteriophages/ml) were added to the culture and the resulting solutions were kept at 37° C for 6-8 hours.

At the end of this period, the cells were separates from the culture medium by centrifuging and the supernatant liquids recovered. 0.2 ml of a 20% solution of polyethyleneglycol (PEG) and 2.5 m NaCl was added to 1 ml of the supernatant liquid to precipitate the bacteriophages.

After 15 minutes at ambient temperature (20-25° C) the mixture was centrifuged at (12,000 g for 5 minutes) in an Eppendorf centrifuge at 20° C and the bacteriophages thus recovered were resuspended in 100 µl of TE buffer (Tris-HCl 10 mM pH 7.5, 1 mM ETA).

The solution was then extracted once with one volume of saturated phenol with water and twice with ethyl ether and finally, the single stranded DNA was precipitated by the addition of 250 µl of ethanol an 10 µl of 3M ammonium acetate to the aqueous phase.

The DNA was separated from the reaction mixture by centrifuging, resuspended in 20µl of TE buffer and used for site directed mutagenesis (Zoller et al. DNA 3, 479-488, 1984).

For this purpose, oligonucleotides were synthesised by means of an automatic System 1 Plus DNA synthesizer system (Beckman), in which the bases which code for at least one of the aminoacids which are of interest, are modified so as to code for another aminoacid. The oligonucleotides, which are complementary to the sequence present in the single stranded DNA cloned in the plasmid Bluescript SK, are used as primers for the DNA polymerase which copies the whole nucleotide sequence of the Bluescript incorporating the mutations present in the primer.

In practice, 2 µl of 10 mM ATP, 2 µl of Kinase buffer 10 x (500 mM Tris-HCl pH 8.0, 100 mM $MgCl_2$), 1µl of dithiothreitol (DTT) 100 mM and 5 U of Kinase polynucleotide (Boehringer) were added to 3 moles of the synthetic oligonucleotides and the final volume brought to 20 µl. Ihe mixture was incubated at 37° C for 30 minutes and the enzyme rendered inactive at 70° C for 10 minutes. 1 µg of the single strand DNA used as a template, 1 µl of 1 mM Tris-Hcl pH 8.0 buffer and 10 mm of $MgCl_2$ in one volume of 10 x kinase, were added to 2 ul of the primer. Ihe mixture was kept at 80° C for approximately 3 minutes and then at an ambient temperature for approximately 1 hour.

10 µl of a buffer of 1 mM Tris-HCl, pH 8.0, 10 mM $MgCl_2$, 0.05 mM ATP, 1 mM DTT, 0.5 mM of the four desoxynucleotides. 1 U of T4 DNA ligase and 2.5 U of DNA polymerase I (Klenow Fragment) were then added. The mixture was then incubated at 15° C for one night and then used to transform E. coli JM 101 cells as described above.

The plasmids containing the mutated S1 gene were then identified by means of the hybridisation technique with the use of the primer used for the mutagenesis, marked with $P^{32}$ as the probe. In practice nitrocellulose filters containing the transformed colonies were hybridised in 6xSSC (1xSCC=0.015 M NaCl, 0.015M trisodium citrate, pH 7), 10xDenhardt (1% BSA, 1% Ficoll, 1% polyvinyl pyrrolidone) and 2% sodium dodecylsulphate (DSD) at 20-25° C for 18 hours and then washed for 2 hours in 6xSSC at the following temperatures: mutants 25 and 26 (45° C), mutants 28, 22 and 29 (48° C), 27 (54° C), mutants 31 and 41 (46° C). The mutations were confirmed by the analysis of the nucleotide sequence of the gene by the method of Sanger F. et al. (PNAS 74, 5463, 1977). By means of the operation described above, the plasmids were prepared, containing the gene which codes for S1, modified as follows:

41: Tyrosine 8 and arginine 9 were replaced respectively by aspartic acid and glycine, with the use of the primer GTCATAGCCGTCTACGGT.

The corresponding gene was modified thus:

620-CGCCACCGTATACCGCTATGACTCCCGCCCG-650
620-CGCCACCGTAGACGGCTATGACTCCCGCCCG-650

22: Phenylalanine 50 and threonine 53 were replaced respectively by glutamic acid and isoleucine with the use of the primer TGGAGACGTCAGCGCTGT. The corresponding gene was modified thus:
The sequence 750-AGCGCTTTCGTCTCCACCAGC-770 was changed into 750-AGCGCTGACGTCTCCATCAGC-770.

25: Glycine 99 was replaced by glutamic acid with the use of the primer CTGGCGGCTTCGTAGAAA;
The corresponding gene was modified thus:
the sequence 910-TACGGCGCCGC-920 was changed into 910-TACGAAGCCGC-920.

17: Aspartic acid 109 was replaced by glycine with the use of the primer CTGGTAGGTGTCCAGCGCGCC.
The corresponding gene was modified thus:
the sequence 930-GTCGACACTTA-940 was changed into 930-GTCGGCACTTA-940.

27: Glycine 121 was replaced by glutamic acid with the use of the primer GCCAGCGCTTCGGCGAGG.
The corresponding gene was modified thus:
the sequence 956-GCCGGCGCGCT-966 was changed into 956-GCCGAAGCGCT-966.

16: Alanine in position 124 was replaced by aspartic acid with the use of the primer GCCATAAGTGCCGACGTATTC
The corresponding gene was modified thus:
the sequence 976-TGGCCACCTAC-984 was changed into 976-TGGACACCTAC-986.

1716: contains the two mutations 16 and 27 combined.

28: glutamic acid 129 was replaced in glycine with the use of the primer GCCAGATACCCGCTCTGG.
The corresponding gene was modified thus:
the sequence 990-AGCGAATATCT-1000 was changed into 990-AGCGGGTATCT-1000.

29: Arginine 135 was replaced by glutamic acid with the use of the primer GCGGAATGTCCCGGTGTG.
The corresponding gene was modified thus:
the sequence 1010-GCGCATTCCGC-1020 was changed into 1010-GGACATTCCGC-1020.

31: Threonine 159 was replaced by lysine with the use of the primer TACTCCGTTTTCGTGGTC.
The corresponding gene was rodified thus:
1070-GCATCACCGGCGAGACCACGACCACGGAGTA-1090 was changed into 1070-GCATCACCGGCGAGACCACGAAAACGGAGTA-1090.

26. Tyrosine 111 was replaced by glycine. Moreover, because of a partial duplication of a fragment of the primer, the aminoacids Aps Thr Gly Gly were inserted in position 113 with the use of the primer CGCCACCAGTGCGACGTATTCGA.
The corresponding gene was modified thus:
930-GTCGACACTTATGGCGACAAT-950
930-GTCGACACTGGTGGCGACACTGGTGGCGACAAT-950

The plasmids containing the S1 gene were digested again with EcoRI and HindIII restrictions enzymes and the DNA fragments containing the mutations described above were separated from the digestion mixture by means of gel electrophoresis, electroeluted and cloned in the PEx-34B vector in the ligation mixture as described above.

The ligation mixtures were used to transform competent E. coli K12-ΔH1-Δtrp cells and the transformants isolated on LB agar medium with the addition of 30 ug/ml of ampicillin at 30 °C.

The positive clones containing the mutated plasmids were then grown in a liquid LB medium as described in Example 2 above, and after cell lysing, the immunoreactivity of the S1 subunits thus produced towards L10 MAb was determined. The results are given in Figure 2.

## Claims

1. An immunologically-active peptide capable of inducing in man, both alone and bound to a macromolecular support, the formation of antibodies which are able to recognise and neutralise pertussis toxin produced by a strain of Bordetella pertussis, where the peptide is characterised in that it contains, in the correct arrangment and in non-contiguous positions, the regions A, B and C of the S1 subunit situated between aminoacids 1-20, 40-80 and 123-180 respectively.

2. A synthetic peptide according to Claim 1, comprising the aminoacid sequence 1-235 of the S1 subunit of pertussis toxin in which the cysteines in positions 41 and 201 are linked by a disulphide bridge.

3. A peptide according to Claim 1 obtained by means of chemical synthesis.

4. A peptide according to Claim 1, obtained by means of a method which includes the growth in a suitable culture medium, of a micro-organism transformed by a plasmid containing the gene which codes for the peptide.

5. A peptide according to Claim 4, in which the micro-organism is Escherichia coli, Bacillus spp. or a yeast.

6. A peptide according to Claim 4, in which the plasmid is PTE 255.

7. A peptide according to Claim 1 obtained by means of a method which includes the synthesis of peptides containing at least one of the regions A, B or C of S1, the recombining in vitro of the peptides so that they reassume the correct arrangement and indicate the formation of antibodies which are capable of neutralising native pertussis toxin and finally the separation and purification of the peptide thus obtained.

8. A vaccine against pertussis containing a quantity which is therapeutically effective in man of a peptide according to Claim 1.

9. A vaccine against pertussis according to Claim 8, in which the peptide is bound to an immunogenic macromolecular support.

10. A vaccine against pertussis according to Claim 9, in which the support is a protein or a polysaccharide.

11. A method for inducing immunisation against pertussis which includes the administration of an immunologically effective quantity of the peptide according to Claim 1 and Claim 2, or a vaccine according to Claims 8-10.

12. A diagnositc kit for the determination of pertussis in clinical samples, including a peptide according to Claims 1 and 2.

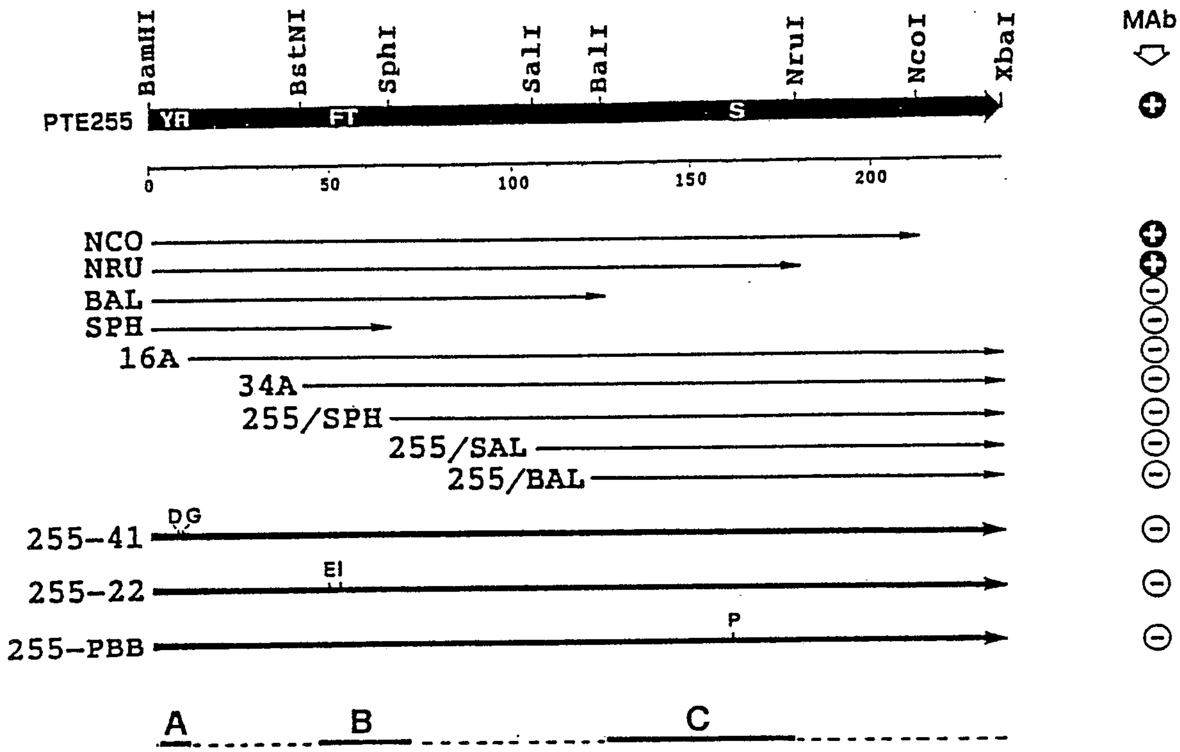


FIGURE 1

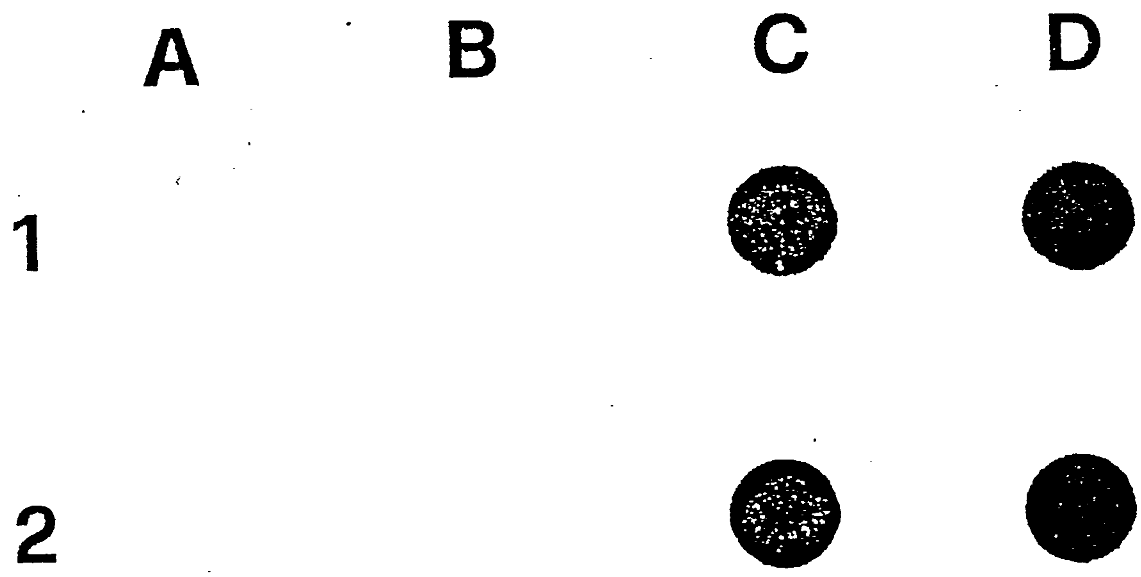


FIGURE 2

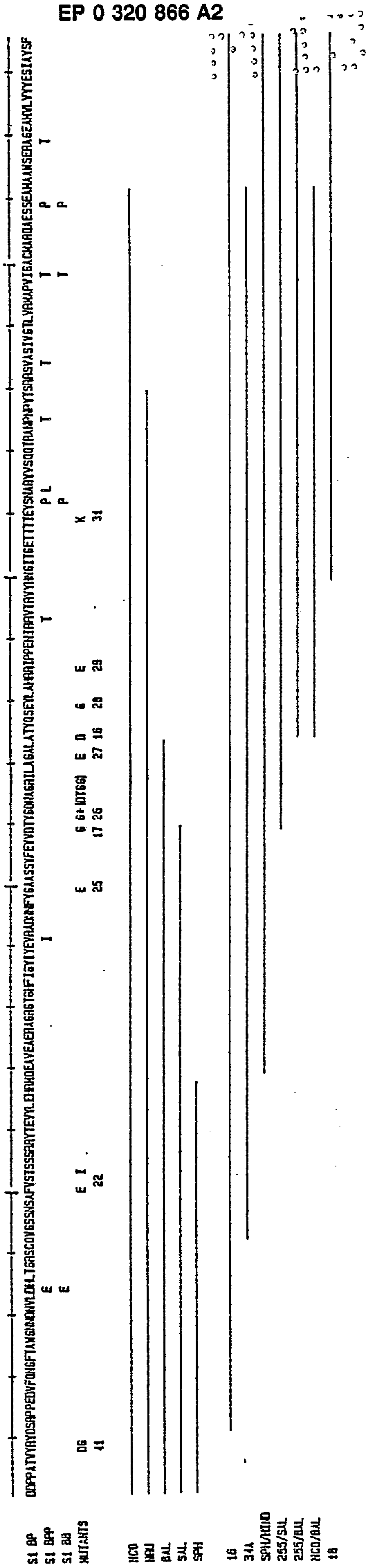
S1 BP
S1 BPP
S1 BB
MUTANTS
NCO
NRU
BAL
SAL
SPH
16
34A
SPH/AUIO
255/SAL
255/BAL
NCO/BAL
18
50
100
150
200
D8
41
22
25
17 26
27 16
28
29
31

FIGURE 3